# EUROPEAN PATENT APPLICATION

(11) **EP 4 811 380 A1**
(43) Date of publication of application: **23.09.2026**
(21) Application number: 26165251.5
(22) Date of filing: 16.03.2026
(51) Int. Cl.: G16H 20/70, G16H 40/63, G16H 50/30, G16H 80/00

(54) **COMMUNICATION SYSTEM, DETECTION CONTROL DEVICE, DETECTION CONTROL METHOD, AND CARRIER MEDIUM**

(30) Priority: 19.03.2025 JP 2025045963
(71) Applicant: Ricoh Company, Ltd., Tokyo 143-8555 (JP)
(72) Inventor: Katoh, Yuuya, Tokyo, 143-8555 (JP); Tanaka, Toshiki, Tokyo, 143-8555 (JP); Murata, Haruki, Tokyo, 143-8555 (JP); Kimura, Yuta, Tokyo, 143-8555 (JP); Watanabe, Shuhei, Tokyo, 143-8555 (JP); Kita, Shinsuke, Tokyo, 143-8555 (JP)
(74) Representative: Marks & Clerk LLP

(57) **Abstract**

A communication system (1) includes a detection control device (6) to perform sensing of a body of a user, and an assistance control device (3) to communicate with the detection control device (6) and control a target device. The detection control device (6) includes a mental state data generation unit (63) to generate mental state data indicating a predetermined mental state of the user based on physical state data indicating a predetermined physical state, the physical state data being obtained based on the sensing of the body of the user, and a transmission unit (60) to transmit the mental state data to the assistance control device (3) based on the mental state data of the user. The assistance control device (3) includes a control unit (34, 37) to control a specific device in a communication space of the user among target devices including the target device based on the mental state data transmitted from the detection control device (6).

## Description

### BACKGROUND

### Technical Field

The present disclosure relates to a technique for assisting user conversations in a communication space, and particularly relates to a communication system, a detection control device, a detection control method, and a carrier medium.

### Related Art

Assistance systems for assisting conversations in communication spaces such as conferences have been proposed. For example, an assistance system has been proposed that uses artificial intelligence (AI) to estimate a situation of the communication space based on the voices and images of participating users, and assist the conversation among the participating users (see Japanese Patent No. 6730843). The assistance system assists the conversation by, for example, outputting recommendation information for divergence or convergence of the conference to a shared display or adjusting the wind direction of an air conditioner in accordance with the content of the estimated situation.

However, such earlier assistance systems, including the system disclosed in Japanese Patent No. 6730843, merely estimate the situation from the voices and images, and thus cannot offer assistance that considers the mental state of a user.

### SUMMARY

The present disclosure described herein provides a communication system including a detection control device to perform sensing of a body of a user, and an assistance control device to communicate with the detection control device and control a target device. The detection control device includes a mental state data generation unit to generate mental state data indicating a predetermined mental state of the user based on physical state data indicating a predetermined physical state, the physical state data being obtained based on the sensing of the body of the user, and a transmission unit to transmit the mental state data to the assistance control device based on the mental state data of the user. The assistance control device includes a control unit to control a specific device in a communication space of the user among target devices including the target device based on the mental state data transmitted from the detection control device.

The present disclosure described herein provides a detection control device for performing sensing of a body of a user. The device includes a mental state data generation unit to generate mental state data indicating a predetermined mental state of the user based on physical state data indicating a predetermined physical state, the physical state data being obtained based on the sensing of the body of the user, and a transmission unit to transmit the mental state data to an assistance control device that controls a target device based on the mental state data of the user.

The present disclosure described herein provides a detection control method executed by a computer that performs sensing of a body of a user. The method includes generating mental state data indicating a predetermined mental state of the user based on physical state data indicating a predetermined physical state, the physical state data being obtained based on the sensing of the body of the user, and transmitting the mental state data to an assistance control device that controls a target device based on the mental state data of the user.

The present disclosure described herein provides a carrier medium carrying computer-readable code for controlling a computer system to carry out a detection control method. The method includes generating mental state data indicating a predetermined mental state of a user based on physical state data indicating a predetermined physical state, the physical state data being obtained based on sensing of a body of the user, and transmitting the mental state data to an assistance control device that controls a target device based on the mental state data of the user.

Accordingly, the assistance control device that controls control targets related to the environment in a communication space to assist a user can grasp the user's mental state based on the user's physical state.

### BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:
FIG. 1 is a schematic diagram of a communication system;
FIG. 2 is a block diagram illustrating a hardware configuration of an assistance control device;
FIG. 3A is a front view of a chair, illustrating a simplified configuration thereof;
FIG. 3B is a side view of the chair, illustrating the simplified configuration thereof;
FIG. 4 is a block diagram illustrating a hardware configuration of a detection control device installed (built) in a chair;
FIG. 5 is a block diagram illustrating functional configurations of an assistance control device and a detection control device according to a first embodiment;
FIG. 6 is a sequence diagram illustrating processes of the assistance control device and the detection control device of a chair according to the first embodiment;
FIG. 7 is a flowchart of a speech recognition process;
FIG. 8 is a flowchart of an output control process;
FIG. 9 is a flowchart of a text generation process;
FIG. 10 is a flowchart of a text generation process;
FIG. 11 is a flowchart of a signal output process;
FIG. 12 is a block diagram illustrating functional configurations of an assistance control device and a detection control device according to a second embodiment;
FIG. 13 is a sequence diagram illustrating processes of the assistance control device and the detection control device of a chair according to the second embodiment;
FIG. 14 is a diagram illustrating functional configurations of an assistance control device and a detection control device according to a third embodiment; and
FIG. 15 is a sequence diagram illustrating processes of the assistance control device and the detection control device of a chair according to the third embodiment.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below.

As used herein, the singular forms "a," "an," and "the" are intended to include the plural forms as well, unless the context clearly indicates otherwise. For the sake of simplicity, identical or similar reference numerals denote identical or similar elements such as parts and materials having the same functions, and redundant descriptions thereof are omitted unless otherwise required.

### First Embodiment

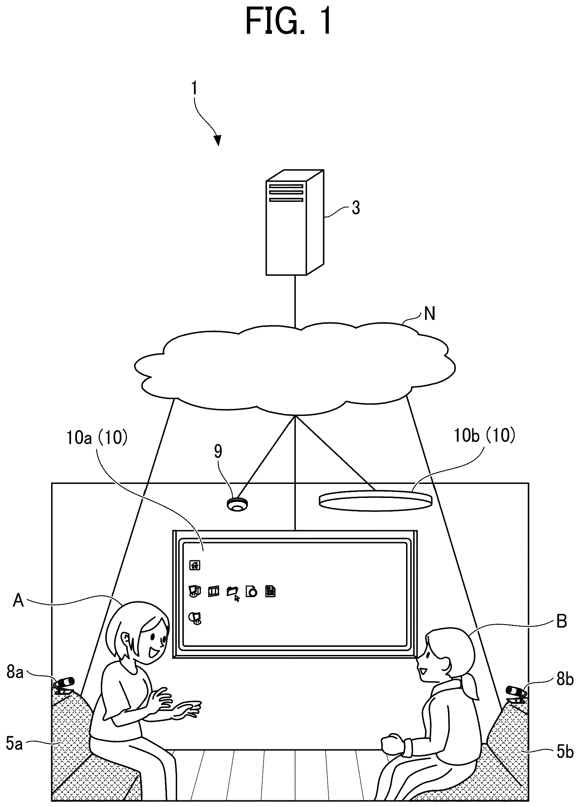

A first embodiment of the present disclosure is described below with reference to FIGS. 1 to 11.

### Overall Configuration of Communication System

An overall configuration of a communication system 1 is described below with reference to FIG. 1. FIG. 1 is a schematic diagram of the communication system 1.

In FIG. 1, chairs 5a and 5b are installed in a space for communication in a certain room. The chairs 5a and 5b are provided with directional microphones 8a and 8b, respectively.

The microphone 8a can collect a voice of a user A seated on the chair 5a. The microphone 8b can collect a voice of a user B seated on the chair 5b.

The chairs 5a and 5b are collectively referred to as "chair 5." The microphones 8a and 8b are collectively referred to as "microphone 8." The chair 5 is provided with sensors 7, details of which will be described later.

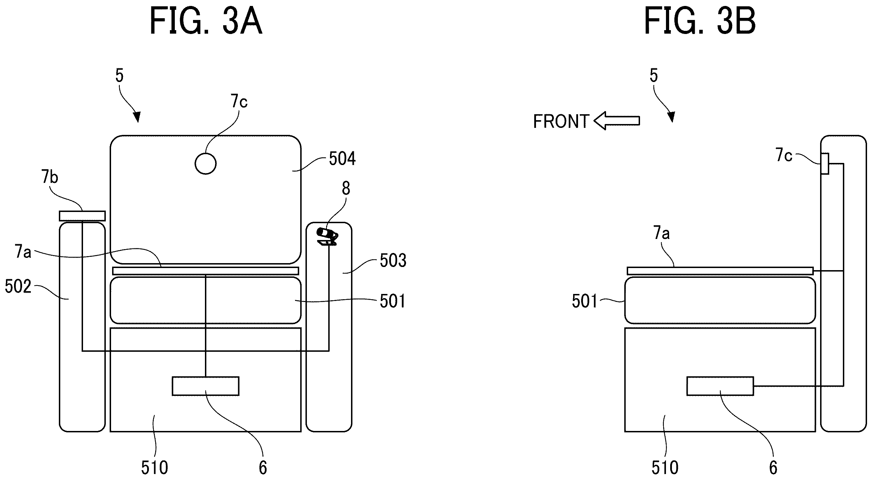

A speaker 9 is installed on the ceiling of the room. A display 10a is installed on a wall of the room. An illumination device 10b is installed on the ceiling of the room. As illustrated in FIGS. 3A and 3B, the chair 5 is provided with a detection control device 6.

An assistance control device 3 is a computer that controls assistance for communication of a user. The assistance control device 3 can communicate with the chair 5, the speaker 9, the display 10a, and the illumination device 10b via a communication network N such as the Internet or a local area network (LAN).

The display 10a and the illumination device 10b are examples of a control target 10.

### Hardware Configuration

### Hardware Configuration of Assistance Control Device

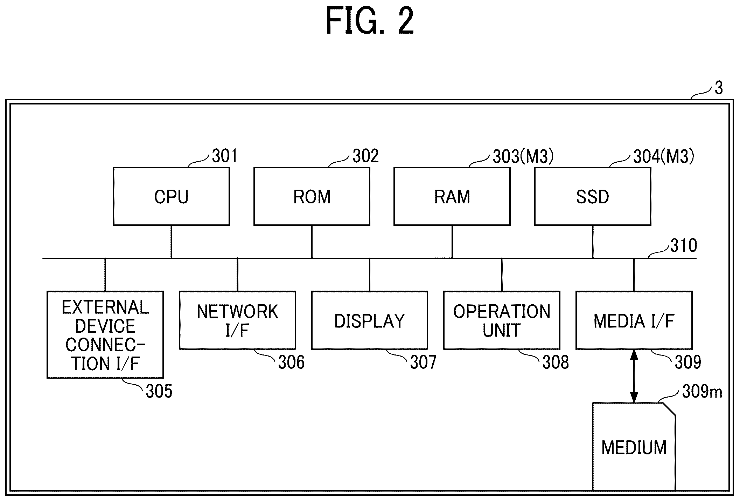

A hardware configuration of the assistance control device 3 is described below with reference to FIG. 2. FIG. 2 is a diagram of a hardware configuration of the assistance control device 3.

As illustrated in FIG. 2, the assistance control device 3 includes, as a computer, a central processing unit (CPU) 301, a read-only memory (ROM) 302, a random-access memory (RAM) 303, a solid-state drive (SSD) 304, an external device connection interface (I/F) 305, a network I/F 306, a display 307, an operation unit 308, a media I/F 309, and a bus line 310.

The CPU 301 controls the overall operation of the assistance control device 3. The ROM 302 stores a program used for booting the CPU 301, such as an initial program loader (IPL). The RAM 303 is used as a work area for the CPU 301.

The SSD 304 reads or writes various types of data under the control of the CPU 301. The assistance control device 3 may include a hard disk drive (HDD) instead of the SSD 304.

The external device connection I/F 305 is an interface for connection with various external devices. The external devices in this case include, but are not limited to, a display, a speaker, a keyboard, a mouse, a universal serial bus (USB) memory, and a printer.

The network I/F 306 is an interface for performing data communication via the communication network N.

The display 307 is an example of a display unit that displays various images, and may be, for example, a liquid crystal display (LCD) or an organic electroluminescent display.

The operation unit 308 functions as an input device that performs operations such as selecting and executing instructions, selecting processing targets, and moving a cursor, and includes various operation buttons, a power switch, a shutter button, and a touch panel.

The media I/F 309 controls the reading of data from, or the writing (storing) of data to (in), a recording medium 309m such as a flash memory. Examples of the recording medium 309m include, but are not limited to, a digital versatile disc (DVD) and a Blu-ray Disc^{®}.

The bus line 310 is, for example, an address bus or a data bus, which electrically connects the components or elements such as the CPU 301.

### Configuration of Chair

A simplified configuration of the chair 5 is described below with reference to FIGS. 3A and 3B. FIG. 3A is a front view of the chair 5, illustrating a simplified configuration thereof, and FIG. 3B is a side view of the chair 5, illustrating the simplified configuration thereof.

As illustrated in FIGS. 3A and 3B, the chair 5 includes a seat 501, armrests 502 and 503 on both sides of the seat 501, and a backrest 504. A storage 510 is provided below the seat 501. The detection control device 6 is housed within the storage 510.

A pressure sensor 7a is disposed on an upper portion of the seat 501. The pressure sensor 7a detects the manner in which the user is seated. A contact sensor 7b is disposed on an upper portion of the armrest 502. The contact sensor 7b detects contact by a finger or hand of the user. An upper portion of the backrest 504 includes a respiration sensor 7c that detects displacement produced by a change in the volume of the chest or abdomen during a respiration cycle (inhalation or exhalation) of the user. The respiration sensor 7c can detect a physical state such as heavy breathing of the user.

The pressure sensor 7a, the contact sensor 7b, and the respiration sensor 7c are examples of the sensor 7.

The microphone 8 is disposed outside or inside the armrest 503. The speaker 9 may be disposed on or in the chair 5, the floor, or a table instead of being located on the ceiling as illustrated in FIG. 1.

The detection control device 6 is electrically connected to each sensor 7 and the microphone 8 through, for example, a cord.

### Hardware Configuration of Detection Control Device

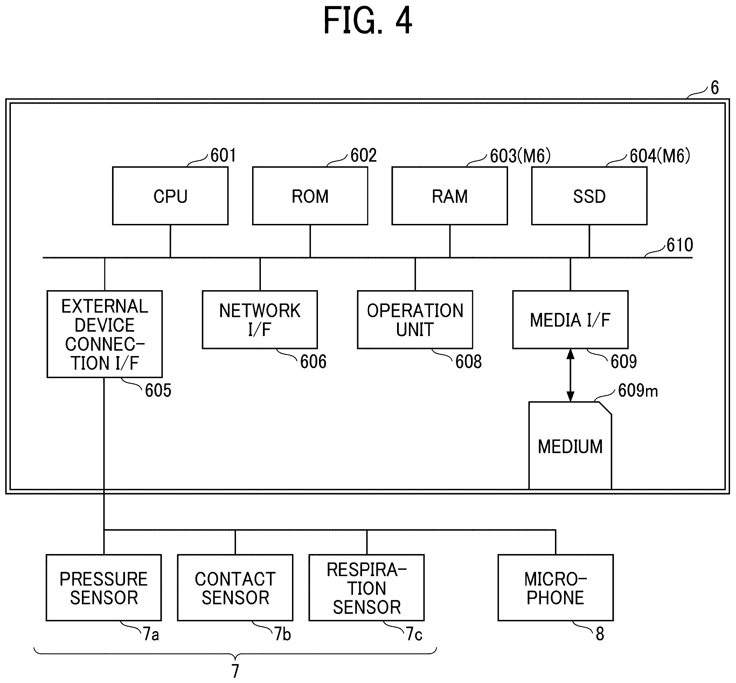

A hardware configuration of the detection control device 6 is described below with reference to FIG. 4. FIG. 4 is a block diagram illustrating a hardware configuration of the detection control device 6.

As illustrated in FIG. 4, the detection control device 6 includes, as a computer, a CPU 601, a ROM 602, a RAM 603, an SSD 604, an external device connection I/F 605, a network I/F 606, an operation unit 608, a media I/F 609, and a bus line 610.

The CPU 601 controls the overall operation of the detection control device 6. The ROM 602 stores a program used for booting the CPU 601, such as an IPL. The RAM 603 is used as a work area for the CPU 601.

The SSD 604 reads or writes various types of data under the control of the CPU 601. The detection control device 6 may include an HDD instead of the SSD 604.

The external device connection I/F 605 is an interface for connection with various external devices. The external devices in this case include, but are not limited to, each sensor 7 and the microphone 8.

The network I/F 606 is an interface for performing data communication via the communication network N.

The operation unit 608 functions as an input device that performs operations such as selecting and executing instructions, selecting processing targets, and moving a cursor, and includes various operation buttons, a power switch, a shutter button, and a touch panel.

The media I/F 609 controls the reading of data from, or the writing (storing) of data to (in), a recording medium 609m such as a flash memory. Examples of the recording medium 609m include, but are not limited to, a DVD and a Blu-ray Disc^{®}.

The bus line 610 is, for example, an address bus or a data bus, which electrically connects the components or elements such as the CPU 601.

### Functional Configuration of Communication System

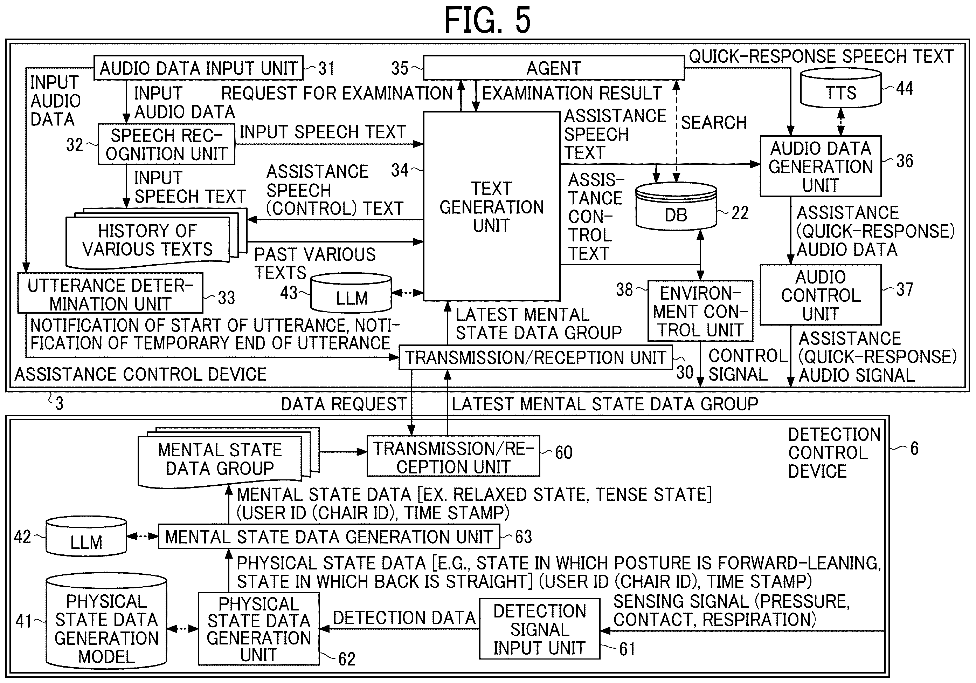

Functional configurations of the assistance control device 3 and the detection control device 6 included in the communication system 1 are described below with reference to FIG. 5. FIG. 5 is a block diagram illustrating functional configurations of the assistance control device 3 and the detection control device 6 according to the first embodiment.

### Functional Configuration of Detection Control Device

The functional configuration of the detection control device 6 is described below. As illustrated in FIG. 5, the detection control device 6 includes a transmission/reception unit 60, a detection signal input unit 61, a physical state data generation unit 62, and a mental state data generation unit 63. Each of these units is a function or means implemented by any of the components illustrated in FIG. 4 operating in accordance with instructions from the CPU 601 based on a program deployed from the SSD 604 to the RAM 603. In the present embodiment, the RAM 603 or the SSD 604 may be referred to as "memory M6." The memory M6 is an example of a storing unit.

A physical state data generation model 41, a large language model (LLM) 42, and a mental state data group are stored in the memory M6.

### Physical State Data Generation Model 41

The physical state data generation model 41 is a trained neural network that receives detection data such as pressure, contact, or respiration as input data, and outputs physical state data indicating a physical state of the user. The physical state data generation model 41 is an example of a trained machine learning model. The physical state indicates, for example, a state in which the posture is forward-leaning, a state in which the back is straight, or a state in which respiration is rough.

### LLM 42

The LLM 42 is a trained neural network that receives the physical state data as input data and outputs mental state data indicating a mental state of the user. Examples of the mental state include a relaxed state and a tense state.

### Transmission/Reception Unit

The transmission/reception unit 60 transmits or receives data to or from external devices of the detection control device 6.

### Detection Signal Input Unit

The detection signal input unit 61 receives analog detection signals (e.g., pressure, contact, and respiration) from the respective sensors 7 that perform sensing of the body of the user and generates digital detection data. The detection signal input unit 61 sequentially outputs the detection data to the physical state data generation unit 62.

### Physical State Data Generation Unit

The physical state data generation unit 62 generates, using the trained physical state data generation model 41, physical state data based on the detection data acquired from the detection signal input unit 61.

### Mental State Data Generation Unit

The mental state data generation unit 63 generates, using the trained LLM 42, mental state data based on the physical state data generated by the physical state data generation unit 62, and stores the mental state data, which includes a user identification (ID) for identifying the user (or a chair ID for identifying the chair) and a time stamp, in the memory M6. The user ID is an example of user identification information. The chair ID is an example of chair identification information for identifying a chair. The user identification information and the chair identification information are examples of "transmission source identification information" for identifying a transmission source or are simply examples of "identification information."

### Functional Configuration of Assistance Control Device

The assistance control device 3 is described below. As illustrated in FIG. 5, the assistance control device 3 includes a transmission/reception unit 30, an audio data input unit 31, a speech recognition unit 32, an utterance determination unit 33, a text generation unit 34, an agent 35, an audio data generation unit 36, an audio control unit 37, and an environment control unit 38. Each of these units is a function or means implemented by any of the components illustrated in FIG. 2 operating in accordance with instructions from the CPU 301 based on a program deployed from the SSD 304 to the RAM 303. In the present embodiment, the RAM 303 or the SSD 304 may be referred to as "memory M3." The text generation unit 34 and the audio control unit 37 define a "control unit."

A database (DB) 22, an LLM 43, a text-to-speech (TTS) 44, and a history of various texts are stored in the memory M3. The various texts include at least one of past input speech text output by the speech recognition unit 32 and past assistance text output by the text generation unit 34. The various texts are used, as past various texts serving as input data, when the text generation unit 34 generates assistance text for assisting communication of the user. The assistance text includes at least one of an assistance speech text and an assistance control text.

The "assistance speech text" indicates content for assisting communication of a user (between users) and includes expressions such as "Why don't you take a break soon?" or "You seem nervous, so please relax."

The "assistance control text" indicates content for controlling the control target 10 as illustrated in FIG. 1 and includes expressions such as "Turning on the display power," and "Increasing the light intensity of the illumination device by one level."

### DB 22

Big data used for search by the agent 35 is stored in the DB 22.

In one example, the DB 22 may be included in an external server instead of the assistance control device 3. In this case, the agent 35 accesses the external server via the transmission/reception unit 30 to search the DB 22, and receives a search result from the external server via the transmission/reception unit 30.

### LLM 43

The LLM 43 is a trained neural network that receives at least the latest mental state data group from the detection control device 6 as input data and outputs assistance text as output data.

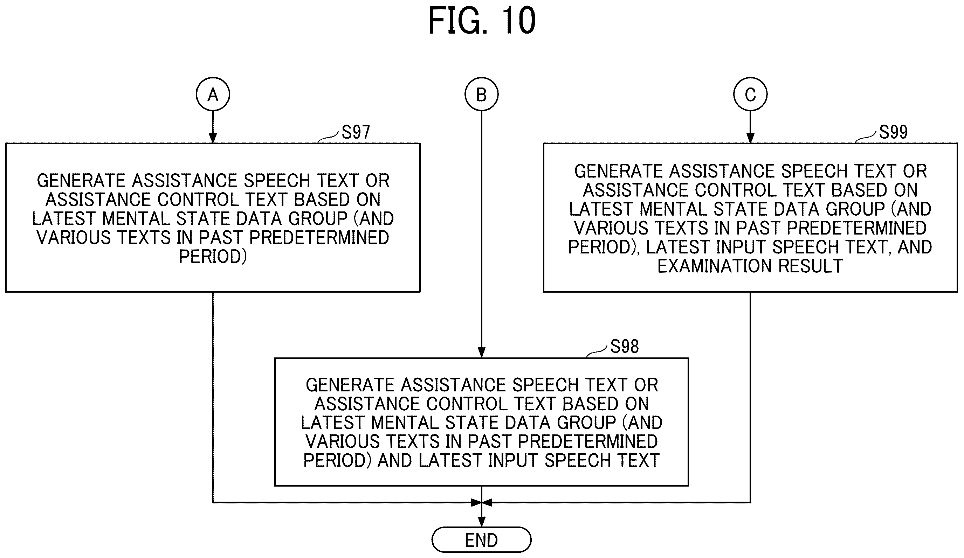

The input data includes, in addition to the latest mental state data group, at least one of various texts in a past predetermined period acquired (input) from the memory M3, the latest input speech text acquired (input) from the speech recognition unit 32, and an examination result acquired (input) from the agent 35. This will be described in detail later with reference to FIG. 10.

### TTS 44

The TTS 44 is a trained neural network that receives text (characters) as input data and outputs data of human-like natural speech (voice data).

### Transmission/Reception Unit

The transmission/reception unit 30 transmits or receives data to or from external devices of the assistance control device 3.

### Audio Data Input Unit

The audio data input unit 31 acquires input audio data from the detection control device 6, and outputs the input audio data to the speech recognition unit 32 and the utterance determination unit 33.

### Speech Recognition Unit

The speech recognition unit 32 performs speech recognition based on the input audio data acquired from the audio data input unit 31, generates the latest input speech text, outputs the latest input speech text to the text generation unit 34, and stores the latest input speech text in the memory M3.

### Utterance Determination Unit

The utterance determination unit 33 determines an utterance period (including a start of utterance and a temporary end of utterance) based on the input audio data acquired from the audio data input unit 31. The utterance determination unit 33 determines that the utterance has temporarily ended in the case of silence for a predetermined time period (for example, three seconds). The utterance determination unit 33 determines that the utterance has started when the utterance has been made again.

### Text Generation Unit

The text generation unit 34 generates, using the LLM 43, assistance speech text or assistance control text based on at least the latest mental state data group of the user.

In this case, basically, the text generation unit 34 generates assistance text based on a time-series change in each item of mental state data included in a first mental state data group of predetermined mental state data stored (accumulated) in the memory M6 within a first predetermined time period (for example, one minute). When the utterance determination unit 33 determines the start of utterance of the user within the first predetermined time period, the text generation unit 34 generates assistance text based on a time-series change in each item of mental state data included in a second mental state data group of predetermined mental state data stored (accumulated) in the memory M6 within a second predetermined time period (for example, 30 seconds) from a time point at which the first predetermined time period begins to a time point at which the start of utterance is determined.

For example, when the mental state of the user is a "tense state," the text generation unit 34 generates assistance speech text indicating "Please relax" or generates assistance control text for changing "the illumination light of illumination device to a warm-light color."

In addition to the predetermined mental state data, the text generation unit 34 generates the assistance text based on at least one of the latest input speech text based on the utterance of the user, the past input speech text output by the speech recognition unit 32, the past assistance text generated by the text generation unit 34, and the examination result acquired by the agent 35. Specific processes are described below.

The text generation unit 34 can generate, using the LLM 43, the assistance speech text or the assistance control text in consideration of the flow (context) of a conversation until then based on various texts (at least one of the past input speech text and the past assistance text) in a past predetermined period serving as a history of various texts.

The history of various texts includes the same input speech text stored in the memory M3 when the speech recognition unit 32 inputs the input speech text to the text generation unit 34. The history of various texts also includes the assistance speech text stored in the memory M3 when the text generation unit 34 inputs the assistance speech text to the audio data generation unit 36 and the DB 22. The history of various texts further includes the assistance control text stored in the memory M3 when the text generation unit 34 inputs the assistance control text to the environment control unit 38 and the DB 22.

The text generation unit 34 can generate, using the LLM 43, the assistance speech text or the assistance control text based on the latest input speech text acquired from the speech recognition unit 32 in consideration of the content of utterance of the user.

The text generation unit 34 can generate, using the LLM 43, the assistance speech text or the assistance control text based on the examination result acquired from the agent 35 in consideration of the examination result. Prior to this, the text generation unit 34 outputs a request for examination to the agent 35. The request for examination is the content of conversation such as "please investigate ..." rather than the content of conversation such as a discussion.

Accordingly, the text generation unit 34 can the generate the assistance speech text or the assistance control text when receiving at least the latest mental state data group among the latest mental state data group, the past various texts, the input speech text, and the examination result. Further, the text generation unit 34 can generate the assistance speech text or the assistance control text when receiving at least one of the past various texts, the input speech text, and the examination result in addition to the latest mental state data group.

### Agent

The agent 35 indicates an artificial intelligence (AI) agent and executes tasks through interaction with a user to achieve specific objectives.

For example, when the agent 35 acquires a request for examination such as "please investigate ..." from the text generation unit 34, the agent 35 outputs an examination result to the text generation unit 34 by searching the DB 22 or by performing the examination by itself without searching the DB 22.

The agent 35 outputs a quick-response speech text to the audio data generation unit 36. The content of the quick-response speech text includes expressions such as "It is under examination. Please wait for a while." The content is finally output from the speaker 9. Thus, the user can recognize that the assistance control device 3 is not neglecting the examination but is actually in the middle of the examination.

### Audio Data Generation Unit

The audio data generation unit 36 generates, using the TTS 44, assistance audio data based on the assistance speech text acquired from the text generation unit 34. The audio data generation unit 36 generates, using the TTS 44, quick-response audio data based on the quick-response speech text acquired from the agent 35. The process performed by the audio data generation unit 36 may be referred to as "speech generation," "speech synthesis," or "speech reading."

### Audio Control Unit

The audio control unit 37 generates an analog assistance audio signal or an analog quick-response audio signal from digital audio data, and transmits (outputs) the analog assistance audio signal or the analog quick-response audio signal to the speaker 9 in the space for communication to control output of assistance audio by the speaker 9. Accordingly, audio for assisting communication of the user, such as "Please relax," is output from the speaker 9 illustrated in FIG. 1.

When respective speakers 9 are installed in multiple rooms that are multiple spaces for communication, the speaker IDs for identifying the speakers 9 and the chair IDs of the chairs 5 included in the mental state (physical state) data are stored in the memory M3 in association with each other. Accordingly, the audio control unit 37 identifies the speaker 9 that serves as the transmission destination with reference to the chair ID included in the latest environmental state data group. When the user performs a process of associating his/her user ID with the chair ID associated with the speaker ID and stores the user ID in the memory M3, the assistance control device 3 can identify the speaker 9 that serves as the transmission destination and the user with reference to the user ID included in the latest environmental state data group. The speaker ID is an example of speaker identification information.

### Environment Control Unit

The environment control unit 38 generates an analog control signal from the assistance control text acquired from the text generation unit 34 and transmits (outputs) the analog control signal to the control target 10 to control the control target 10 relating to the environment in the space for communication. Accordingly, the control target 10 illustrated in FIG. 1 is remotely controlled, and as a result, the conversation of the user can be assisted.

When respective control targets 10 are installed in multiple rooms that are multiple spaces for communication, the control targets 10 and the chair IDs of the chairs 5 are stored in the memory M3 in association with each other. Accordingly, the environment control unit 38 identifies the control target 10 that serves as the transmission destination with reference to the chair ID included in the latest environmental state data group. When the user performs a process of associating his/her user ID with the chair ID associated with the speaker ID and stores the user ID in the memory M3, the assistance control device 3 can identify the control target 10 that serves as the transmission destination and the user with reference to the user ID included in the latest environmental state group.

### User State Estimation Process

In step S1, the detection signal input unit 61 acquires detection signals (e.g., pressure, contact, and respiration) from the respective sensors 7 and generates detection data. The detection signal input unit 61 sequentially outputs the detection data to the physical state data generation unit 62.

In step S2, the physical state data generation unit 62 generates, using the trained physical state data generation model 41, physical state data based on the detection data acquired from the detection signal input unit 61.

The physical state data indicates, for example, a probability of a state in which the posture is forward-leaning, or a probability of a state in which the back is straight. The physical state data generation unit 62 outputs the physical state data, which includes the user ID of the user A (or the chair ID of the chair 5a) and the time stamp indicating the time of the acquisition of the detection signals, to the mental state data generation unit 63.

In step S3, the mental state data generation unit 63 generates, using the trained LLM 42, mental state data indicating the relaxed state or another posture-related state based on the physical state data generated by the physical state data generation unit 62, and stores the mental state data, which includes the user ID (or the chair ID) and the time stamp, in the memory M6. For example, the mental state data generation unit 63 can estimate that the user is in a relaxed state when the probability of the state in which the posture is forward-leaning is relatively high, and can estimate that the user is in a tense state when the probability of the state in which the back is straight is relatively high.

The above steps S1 to S3 are repeated as occasion arises unless the conversation by the communication system 1 ends. Accordingly, unless the conversation by the communication system 1 ends, the mental state data is accumulated in time series in the RAM 603.

In step S4a, the transmission/reception unit 60 of the detection control device 6 converts an analog audio signal collected by the microphone 8 into digital audio data and transmits the digital audio data to the assistance control device 3 as occasion arises.

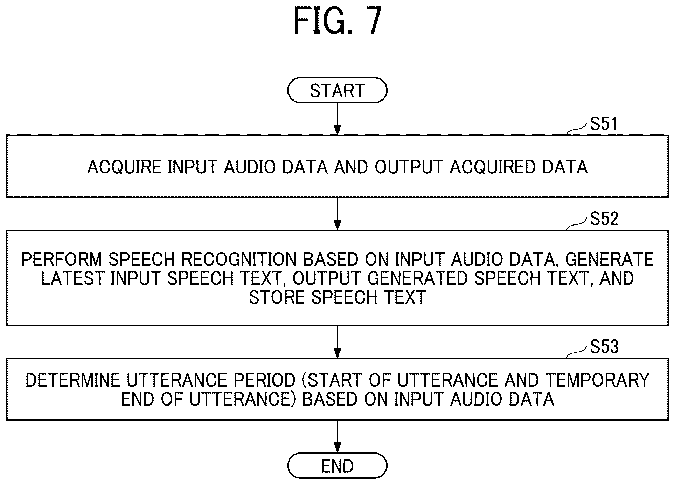

In step S5, the assistance control device 3 performs a speech recognition process based on the received audio data. Step S5 is described below in detail with reference to FIG. 7. FIG. 7 is a flowchart of the speech recognition process.

In step S51, the audio data input unit 31 acquires digital input audio data from the detection control device 6 and outputs the digital input audio data to the speech recognition unit 32 and the utterance determination unit 33.

In step S52, the speech recognition unit 32 performs speech recognition based on the input audio data, generates the latest input speech text, outputs the latest input speech text to the text generation unit 34, and temporarily stores the latest input speech text in the memory M3.

In step S53, the utterance determination unit 33 determines an utterance period (including a start of utterance and a temporary end of utterance) based on the input audio data. The utterance determination unit 33 determines that the utterance has temporarily ended in the case of silence for a predetermined time period. The utterance determination unit 33 determines that the utterance has been started when the utterance has been made again.

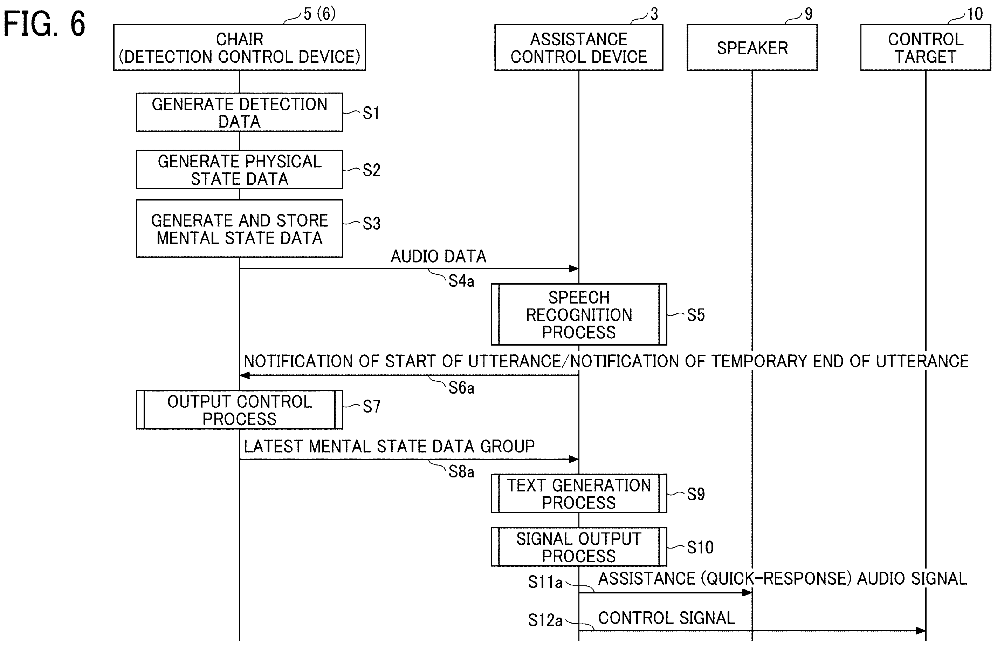

Referring back to FIG. 6, in step S6a, the utterance determination unit 33 transmits a notification of start of utterance or a notification of temporary end of utterance to the detection control device 6 via the transmission/reception unit 30.

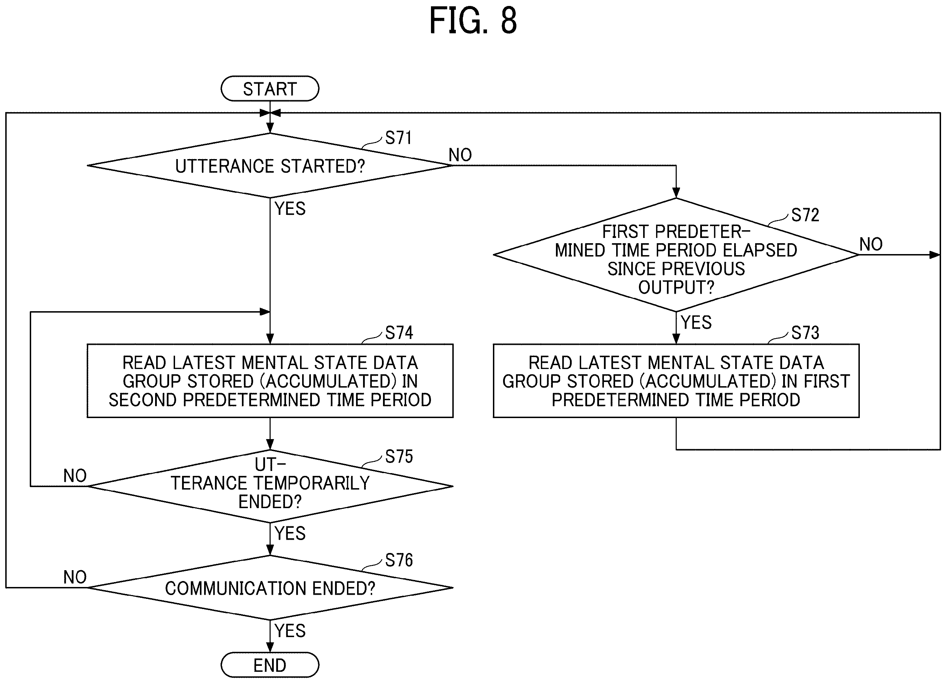

In step S7, the detection control device 6 performs an output control process based on the notification of the start of utterance or the notification of the temporary end of utterance. Step S7 is described below in detail with reference to FIG. 8. FIG. 8 is a flowchart of the output control process.

In step S71, the transmission/reception unit 60 determines whether the notification indicates the start of utterance.

When the notification does not indicate the start of utterance (NO in step S71), in step S72, the transmission/reception unit 60 determines whether a first predetermined time period (for example, one minute) has elapsed from the output of the previous latest mental state data group. When the first predetermined time period has not elapsed (NO in step S72), the process returns to step S71. Thus, the latest mental state data is handled not as a single item but as a group of the latest mental-state data items (the latest mental state data group). The reason is that the text generation unit 34 determines (identifies) the latest mental state of the user based on time-series changes of the multiple latest mental state data items included in the latest mental state data group. In one example, although the accuracy decreases, the text generation unit 34 may determine (identify) the latest mental state of the user based on the latest mental state data alone.

When the first predetermined time period has elapsed (YES in step S72), in step S73, the transmission/reception unit 60 reads the latest mental state data group stored (accumulated) in the first predetermined time period from the memory M6, and the process returns to step S71. In this case, the transmission/reception unit 60 reads the latest mental state data group stored in the first predetermined time period with reference to the time stamp included in the mental state data.

When the notification indicates the start of utterance (YES in step S71), in step S74, the transmission/reception unit 60 reads, from the memory M6, the latest mental state data group stored (accumulated) in a second predetermined time period, which is a period from a time point at which the first predetermined time period begins to a time point at which the start of utterance is determined, and is, for example, 30 seconds. In other words, even when the utterance has not started, the latest mental state data group accumulated in the first predetermined time period is output (see step S73). However, when the utterance has started, the latest mental state data group stored (accumulated) in the memory M6 until then is output without waiting for the first predetermined time period (see step S74). In this case, the transmission/reception unit 60 reads the latest mental state data group stored in the second predetermined time period with reference to the time stamp included in the mental state data.

In step S75, the transmission/reception unit 60 determines whether the notification of the temporary end of utterance has been received. When the notification of the temporary end of utterance has not been received (NO in step S75), step S74 is repeated.

When the transmission/reception unit 60 determines that the notification of the temporary end of utterance has been received (YES in step S75), in step S76, the transmission/reception unit 60 determines whether the conversation (communication) has ended by, for example, an operation performed by the user on the detection control device 6. When the conversation has not ended (NO in step S76), the process returns to step S71. In contrast, when the conversation has ended (YES in step S76), the assistance for communication using the detection control device 6 ends.

Referring back to FIG. 6, in step S8a, the transmission/reception unit 60 transmits the latest mental state data group output in step S73 or step S74 to the assistance control device 3. Accordingly, the transmission/reception unit 30 receives the latest mental state data group.

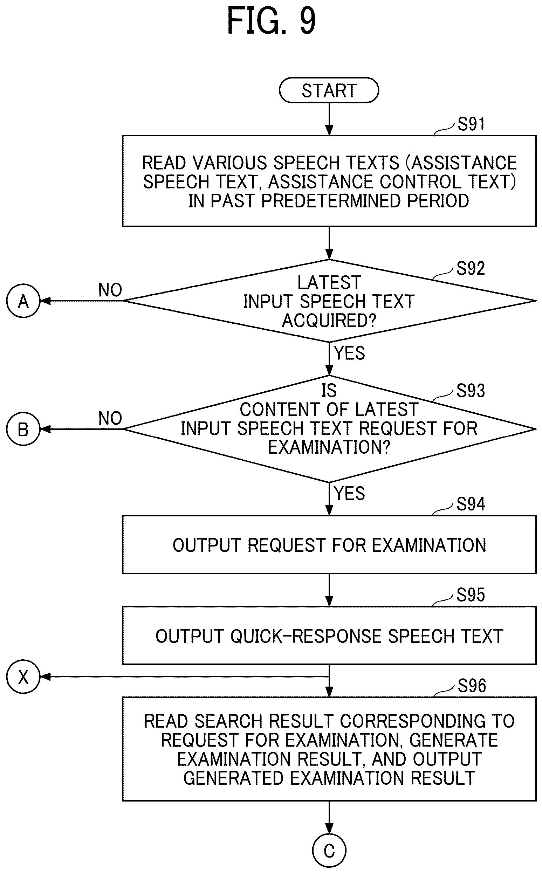

In step S9, the assistance control device 3 performs a text generation process. Step S9 is described below in detail with reference to FIGS. 9 and 10. FIGS. 9 and 10 are flowcharts presenting the text generation process.

In step S91, the text generation unit 34 reads various speech texts (at least one of an assistance speech text and an assistance control text) in a past predetermined period from the memory M3.

In step S92, the text generation unit 34 determines whether the text generation unit 34 has acquired the latest input speech text from the speech recognition unit 32. When the text generation unit 34 has not acquired the latest input speech text from the speech recognition unit 32 (NO in step S92), the text generation unit 34 recognizes a silent state, and the process proceeds to step S97 described later.

When the text generation unit 34 has acquired the latest input speech text from the speech recognition unit 32 (YES in step S92), in step S93, the text generation unit 34 determines whether the content of the latest input speech text is a request for examination such as "please investigate ...." When the content is not the request for examination (NO in step S93), the process proceeds to step S98 described later.

When the content is the request for examination (YES in step S93), in step S94, the text generation unit 34 outputs data indicating the request for examination to the agent 35.

In step S95, the agent 35 outputs a quick-response speech text to the audio data generation unit 36. The content of the quick-response speech text includes expressions such as "It is under examination. Please wait for a while."

In step S96, the agent 35 searches the DB 22, reads a search result corresponding to the request for examination, generates an examination result, and outputs the examination result to the text generation unit 34. When the DB 22 is included in an external server of the assistance control device 3, the agent 35 accesses the external server via the transmission/reception unit 30 to search the DB 22, and receives a search result corresponding to the request for examination via the transmission/reception unit 30. In one example, the agent 35 may generate an examination result without using the DB 22.

When the text generation unit 34 has not acquired the latest input speech text from the speech recognition unit 32 (NO in step S92), in step S97, the text generation unit 34 generates, using the LLM 43, an assistance speech text or an assistance control text based on the latest mental state data group (and various texts in a past predetermined period).

In this case, the text generation unit 34 can output assistance audio described later such as "You appear to be tense, so please relax" based on the "latest mental state data group" as input data.

With "various texts in a past predetermined period" as input data, the text generation unit 34 can output an assistance audio signal described later in consideration of the flow (context) of the conversation until then. In one example, "various texts in a past predetermined period" does not have to be used as input data.

Although the "latest input speech text" as input data is not used in step S97 unlike steps S98 and S99 described later, the assistance audio described later can be output. For example, even when the conversation between the users stagnates and becomes silent, assistance audio for a suggestion such as "Why don't you take a break now?" is to be output.

When the content is not the request for examination (NO in step S93), in step S98, the text generation unit 34 generates, using the LLM 43, an assistance speech text or an assistance control text based on the latest mental state data group (and the various texts in the past predetermined period) and the latest input speech text. In step S98, the latest input speech text is additionally supplied to the LLM 43 as input data, in contrast to step S97.

After step S96, in step S99, the text generation unit 34 generates, using the LLM 43, an assistance speech text or an assistance control text based on the latest mental state data group (and the various texts in the past predetermined period), the latest input speech text, and the examination result. In step S99, the examination result generated by the agent 35 is additionally supplied to the LLM 43 as input data, in contrast to step S98.

In steps S97 to S99, the various texts in the past predetermined period include at least one of the past input speech text stored by the speech recognition unit 32 and the past assistance text stored by the text generation unit 34.

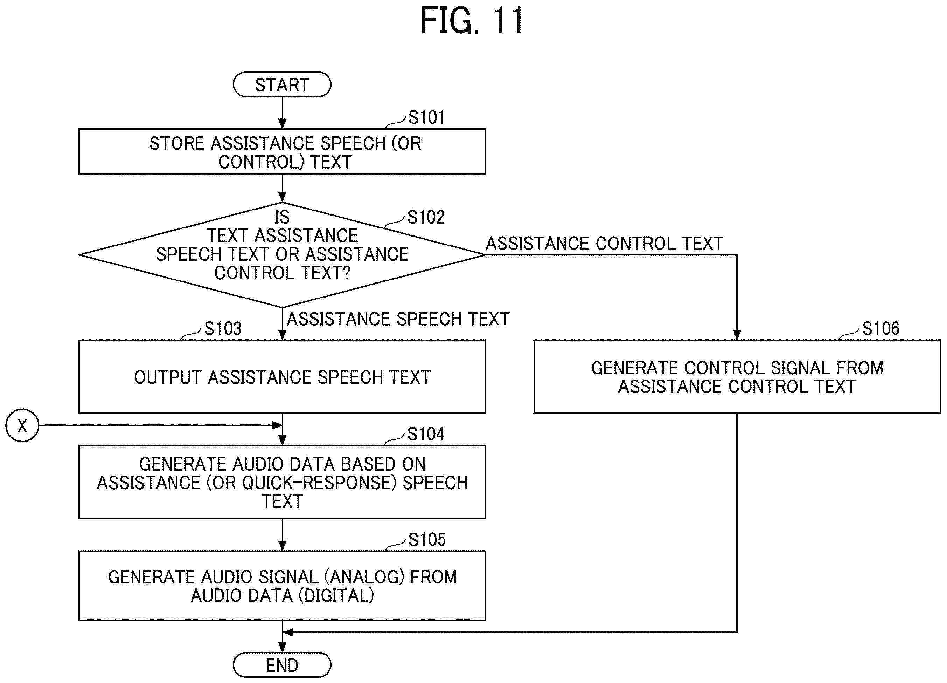

Referring back to FIG. 6, in step S10, the assistance control device 3 performs a signal output process. Step S10 is described below in detail with reference to FIG. 11. FIG. 11 is a flowchart of the signal output process.

In step S101, the text generation unit 34 stores the assistance speech text or the assistance control text in the memory M3 and the DB 22.

In step S102, the text generation unit 34 determines whether the text (the content of the text) is the assistance speech text or the assistance control text.

When the text is the assistance speech text in step S102, in step S103, the text generation unit 34 outputs the assistance speech text to the audio data generation unit 36.

In step S104, the audio data generation unit 36 generates, using the TTS 44, audio data based on the assistance speech text subsequently to step S103 or generates audio data based on the quick-response speech text subsequently to step S95.

In step S105, the audio control unit 37 generates an assistance audio signal or a quick-response audio signal from the audio data.

When the text is the assistance control text in step S102, in step S106, the environment control unit 38 generates a control signal for the control target 10 from the assistance control text.

Referring back to FIG. 6, in step S11a, the audio control unit 37 transmits (outputs) the generated assistance audio signal or quick-response audio signal to the speaker 9. Accordingly, the speaker 9 outputs assistance audio or quick-response audio.

In contrast, in step S12a, the environment control unit 38 identifies the control target 10 from the assistance control text and transmits (outputs) a control signal to the identified control target 10. Accordingly, when the control target 10 is the display 10a, the power can be turned on, and when the control target 10 is the illumination device 10b, the light intensity can be increased by one level.

As described above, according to the first embodiment, for example, in a space for communication as illustrated in FIG. 1, assistance can be offered for a user in consideration of the mental state of the user. In particular, even when the user is in a silent state, assistance can be offered for the user in consideration of the mental state of the user.

Specifically, as illustrated in FIG. 10, the assistance control device 3 can output an assistance audio signal or a control signal in consideration of the mental state of the user by using at least the mental state data group of the user. For example, when the mental state is a "tense state," the assistance control device 3 can output an assistance audio signal for outputting audio indicating "Please relax" or output a control signal for changing "the illumination light of illumination device to a warm-light color."

With the use of the various texts in the past predetermined period, the assistance control device 3 can output an assistance audio signal or a control signal in consideration of the flow (context) of the conversation until then.

Further, with the use of the latest input speech text, the assistance control device 3 can output an assistance audio signal or a control signal in consideration of the content of utterance of the user.

Furthermore, with the use of the examination result generated by the agent 35, the assistance control device 3 can output an assistance audio signal or a control signal in consideration of the examination result.

In contrast, the detection control device 6 estimates the current physical state of the user based on the detection signal (at least one of pressure, contact, and respiration) relating to the user seated on the chair 5, and estimates the mental state from the physical state. The detection control device 6 transmits the latest mental state data group that is the result of the estimation to the assistance control device 3, and thus the latest mental state data group can be used to assist the conversation of the user.

### Second Embodiment

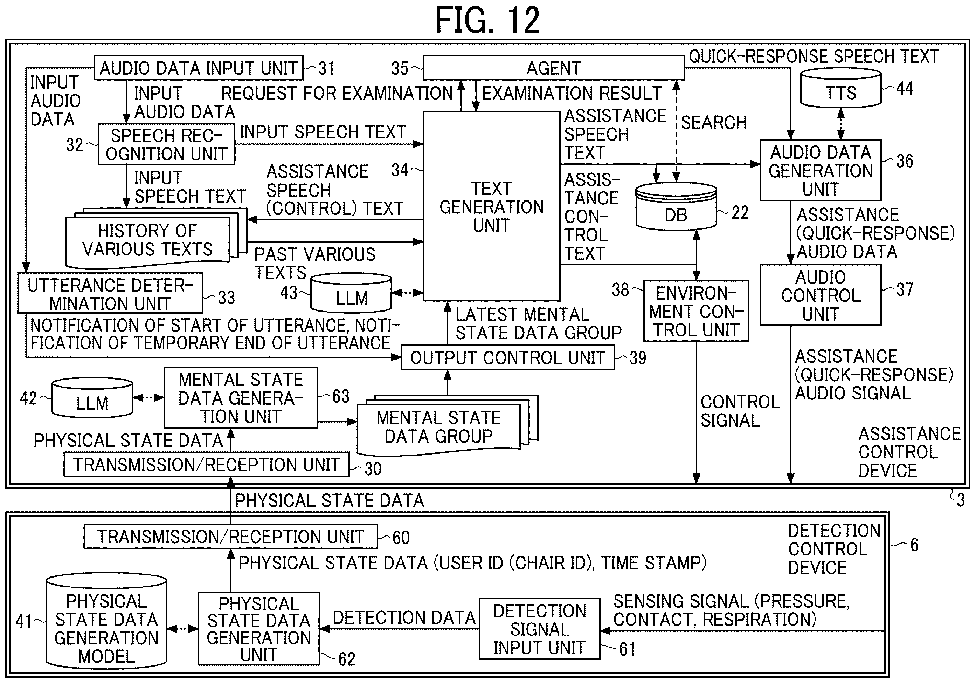

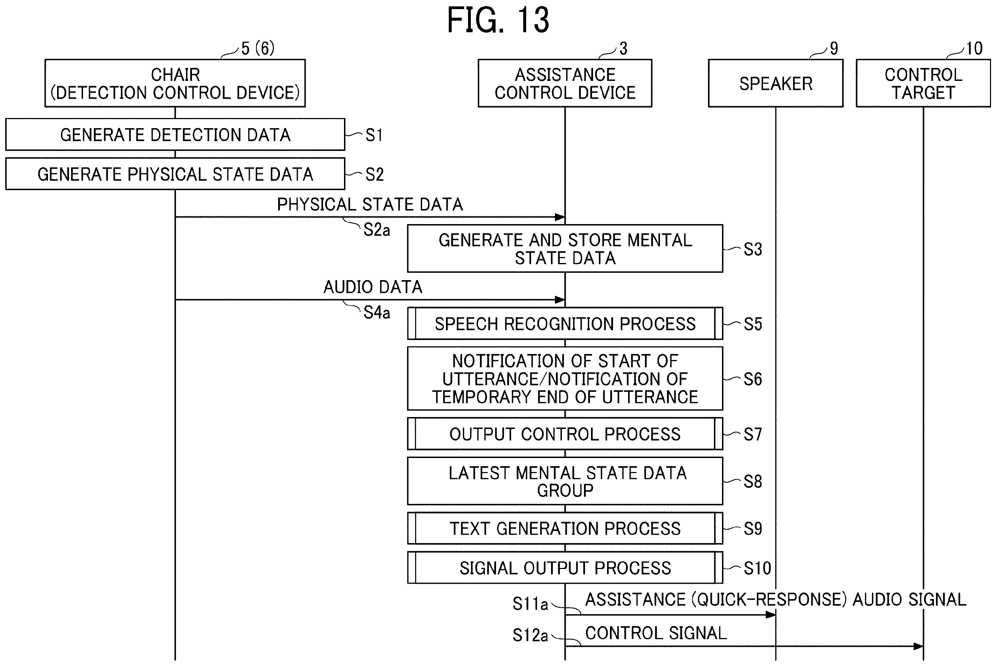

A second embodiment of the present disclosure is described below with reference to FIGS. 12 and 13. FIG. 12 is a block diagram illustrating functional configurations of an assistance control device 3 and a detection control device 6 according to the second embodiment. FIG. 13 is a sequence diagram illustrating processes of the assistance control device 3 and the detection control device 6 of the chair 5 according to the second embodiment.

While the detection control device 6 includes the mental state data generation unit 63 and the LLM 42 in the first embodiment, the assistance control device 3 includes the mental state data generation unit 63 and the LLM 42 in the second embodiment, which is a difference between the first embodiment and the second embodiment. The assistance control device 3 according to the second embodiment further includes an output control unit 39. The output control unit 39 is a function or means implemented by any of the components illustrated in FIG. 2 operating in accordance with instructions from the CPU 301 based on a program deployed from the SSD 304 to the RAM 303. In FIG. 12, identical or similar functional units to those in FIG. 5 are denoted by identical or similar reference signs, and the description thereof will be omitted.

Basically, when the first predetermined time period has elapsed, the output control unit 39 reads the latest mental state data group stored (accumulated) in the first predetermined time period from the memory M3 and outputs the latest mental state data group to the text generation unit 34. When the output control unit39 acquires the notification of the start of utterance from the utterance determination unit 33, the output control unit 39 reads the latest mental state data group stored (accumulated) in the second predetermined time period from the memory M3 and outputs the latest mental state data group to the text generation unit 34.

As illustrated in FIG. 13, in step S2a, the transmission/reception unit 60 transmits the physical state data generated in step S2 to the assistance control device 3. Accordingly, the transmission/reception unit 30 receives the physical state data and outputs the physical state data to the mental state data generation unit 63 of the assistance control device 3.

In step S3, the mental state data generation unit 63 generates, using the trained LLM 42, mental state data based on the physical state data received by the transmission/reception unit 30, and stores the mental state data, which includes the user ID (or the chair ID) and the time stamp, in the memory M3. The subsequent processing is similar to that in the first embodiment, except that steps S5 to S8 are executed inside the assistance control device 3. In step S6, the utterance determination unit 33 transmits a notification of start of utterance or a notification of temporary end of utterance to the output control unit 39. In step S8, the output control unit 39 transmits the latest mental state data group to the text generation unit 34.

While the mental state data group is stored in the memory M6 of the detection control device 6 in the first embodiment, the mental state data group is stored in the memory M3 of the assistance control device 3 in the second embodiment. Thus, in step S74, instead of the transmission/reception unit 60, the output control unit 39 reads the latest mental state data group accumulated in the first or second predetermined time period from the memory M3 with reference to the time stamp included in the mental state data.

As described above, according to the second embodiment, the assistance control device 3 achieves effects similar to those of the first embodiment. Since the assistance control device 3 includes the mental state data generation unit 63 and the LLM 42, these units can be easily upgraded, and the processing load on the detection control device 6 that is a terminal device can be reduced. Thus, the detection control device 6 can be implemented by a relatively inexpensive computer.

### Third Embodiment

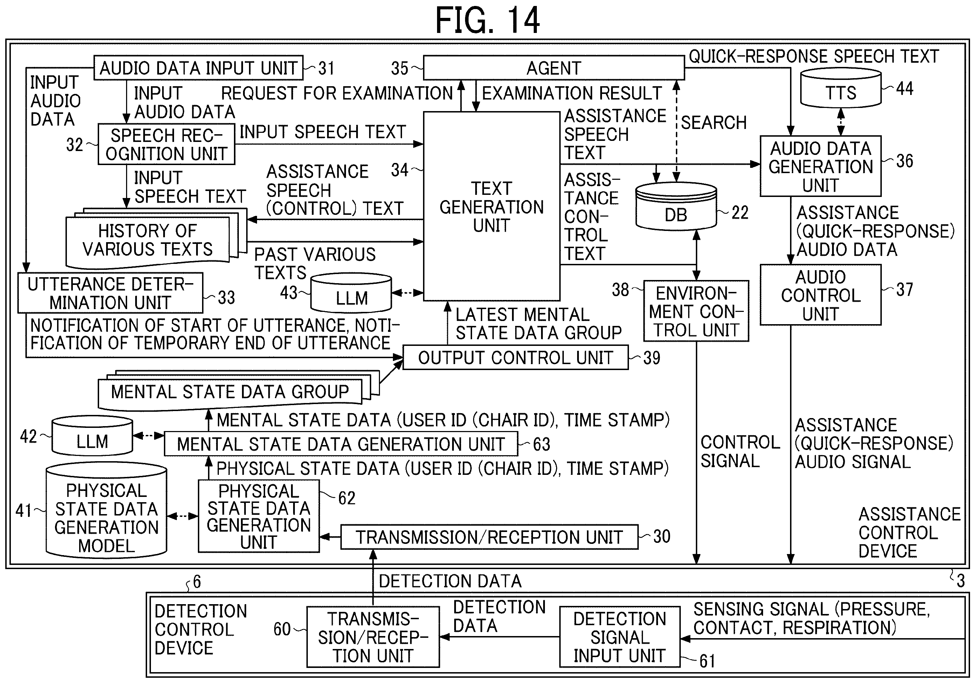

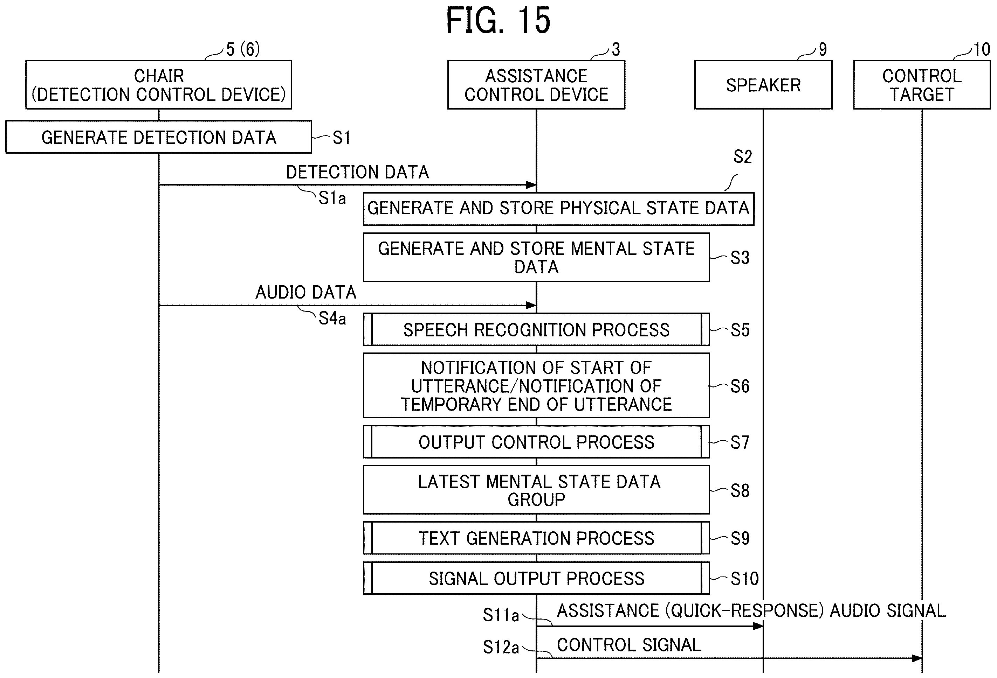

A third embodiment of the present disclosure is described below with reference to FIGS. 14 and 15. FIG. 14 is a diagram illustrating functional configurations of an assistance control device 3 and a detection control device 6 according to the third embodiment. FIG. 15 is a sequence diagram illustrating processes of the assistance control device 3 and the detection control device 6 of the chair 5 according to the third embodiment.

While the detection control device 6 includes the physical state data generation unit 62 and the physical state data generation model 41 in the second embodiment, the assistance control device 3 includes the physical state data generation unit 62 and the physical state data generation model 41 in the third embodiment, which is a difference between the second embodiment and the third embodiment. In FIG. 14, identical or similar functional units as those in FIGS. 5 and 12 are denoted by identical or similar reference numerals, and the description thereof will be omitted.

As illustrated in FIG. 15, in step S1a, the transmission/reception unit 60 transmits the detection data generated in step S1 to the assistance control device 3. Accordingly, the transmission/reception unit 30 receives the detection data and outputs the detection data to the physical state data generation unit 62 of the assistance control device 3.

In step S2, the physical state data generation unit 62 generates, using the trained physical state data generation model 41, physical state data based on the detection data received by the transmission/reception unit 30, and stores the physical state data, which includes the user ID (or the chair ID) and the time stamp, in the memory M3. The subsequent processing is similar to that in the second embodiment.

As described above, according to the third embodiment, the assistance control device 3 achieves effects similar to those of the first embodiment. Since the assistance control device 3 includes the physical state data generation unit 62 and the physical state data generation model 41, these units can be easily upgraded, and the processing load on the detection control device 6 that is a terminal device can be reduced. Thus, the detection control device 6 can be implemented by a further inexpensive computer as compared to the second embodiment.

The above-described embodiments are illustrative and do not limit the present disclosure. Thus, numerous additional modifications and variations are possible in light of the above teachings.
(1) For example, each of the above-described programs can be recorded on a (non-transitory) recording medium and distributed, and may also be provided via the communication network N such as the Internet.
(2) The CPUs 301 and 601, which serve as processors, may each be either a single unit or multiple units.
(3) The sensors 7 disposed on or in the chair 5 may be any one or more of the pressure sensor 7a, the contact (touch) sensor 7b, and the respiration sensor 7c. The sensors 7 include, for example, a human-detection sensor, a temperature sensor, a light sensor, or a sound sensor.
(4) The chair 5 provided with the sensors 7 and the microphone 8 is an example of a smart textile. The smart textile includes a sofa, a bed, a cushion, or an animal-shaped robot such as a cat or a dog, in addition to the chair 5.
(5) The control target 10 includes an air conditioner, a projector, a monitoring camera, or an electric blind, in addition to the display 10a and the illumination device 10b. The control target 10 may include the speaker 9 illustrated in FIG. 1.

Any one of the above-described operations may be performed in various other ways, for example, in an order different from the one described above.

The present invention can be implemented in any convenient form, for example using dedicated hardware, or a mixture of dedicated hardware and software. The present invention may be implemented as computer software implemented by one or more networked processing apparatuses. The processing apparatuses include any suitably programmed apparatuses such as a general purpose computer, a personal digital assistant, a Wireless Application Protocol (WAP) or third-generation (3G)-compliant mobile telephone, and so on. Since the present invention can be implemented as software, each and every aspect of the present invention thus encompasses computer software implementable on a programmable device. The computer software can be provided to the programmable device using any conventional carrier medium (carrier means). The carrier medium includes a transient carrier medium such as an electrical, optical, microwave, acoustic or radio frequency signal carrying the computer code. An example of such a transient medium is a Transmission Control Protocol/Internet Protocol (TCP/IP) signal carrying computer code over an IP network, such as the Internet. The carrier medium may also include a storage medium for storing processor readable code such as a floppy disk, a hard disk, a compact disc-read-only memory (CD-ROM), a magnetic tape device, or a solid state memory device.

## Claims

1. A communication system (1) comprising:
a detection control device (6) configured to perform sensing of a body of a user; and
an assistance control device (3) configured to communicate with the detection control device (6) and control a target device,
the detection control device (6) including:
a mental state data generation unit (63) configured to generate mental state data indicating a predetermined mental state of the user based on physical state data indicating a predetermined physical state, the physical state data being obtained based on the sensing of the body of the user; and
a transmission unit (60) configured to transmit the mental state data to the assistance control device (3) based on the mental state data of the user,
the assistance control device (3) including a control unit (34, 37) configured to control a specific device in a communication space of the user among target devices including the target device based on the mental state data transmitted from the detection control device (6).

2. The communication system (1) according to claim 1,
wherein the detection control device (6) further includes a physical state data generation unit (62) configured to generate, using a trained machine learning model, the physical state data based on detection data obtained through the sensing of the body of the user.

3. The communication system (1) according to claim 2, further comprising a sensor (7) configured to perform the sensing of the body of the user,
wherein the detection control device (6) further includes a detection signal input unit (61) configured to receive a detection signal from the sensor and generate the detection data.

4. The communication system (1) according to claim 3, further comprising a smart textile (5),
wherein the detection control device (6) is disposed at the smart textile (5) together with the sensor (7).

5. The communication system (1) according to claim 4, wherein
the sensor (7) is one of a pressure sensor, a contact sensor, a respiration sensor, a human-detection sensor, a temperature sensor, a light sensor, and a sound sensor, and
the smart textile (5) is one of a chair, a sofa, a bed, a cushion, and an animal-shaped robot.

6. The communication system (1) according to claim 1, wherein
the detection control device (6) further includes a storing unit (M6) configured to store the mental state data, and
the transmission unit (60) configured to transmit, to the assistance control device (3), a first mental state data group of the mental state data stored in the storing unit (M6) within a first predetermined time period.

7. The communication system (1) according to claim 6, wherein
the detection control device (6) further includes a reception unit (60) configured to receive a notification of a start of utterance of the user within the first predetermined time period, and
in response to the reception unit (60) receiving the notification, the transmission unit (60) is configured to transmit, to the assistance control device (3), a second mental state data group of the mental state data stored in the storing unit (M6) within a second predetermined time period from a time point at which the first predetermined time period begins to a time point at which the start of the utterance is determined.

8. A detection control device (6) for performing sensing of a body of a user, the device (6) comprising:
a mental state data generation unit (63) configured to generate mental state data indicating a predetermined mental state of the user based on physical state data indicating a predetermined physical state, the physical state data being obtained based on the sensing of the body of the user; and
a transmission unit (60) configured to transmit the mental state data to an assistance control device that controls a target device based on the mental state data of the user.

9. A detection control method executed by a computer that performs sensing of a body of a user, the method comprising:
generating (S3) mental state data indicating a predetermined mental state of the user based on physical state data indicating a predetermined physical state, the physical state data being obtained based on the sensing of the body of the user; and
transmitting (S8a) the mental state data to an assistance control device that controls a target device based on the mental state data of the user.

10. A carrier medium carrying computer-readable code for controlling a computer system to carry out a detection control method, the method comprising:
generating (S3) mental state data indicating a predetermined mental state of a user based on physical state data indicating a predetermined physical state, the physical state data being obtained based on sensing of a body of the user; and
transmitting (S8a) the mental state data to an assistance control device that controls a target device based on the mental state data of the user.
